# EUROPEAN PATENT APPLICATION

(11) **EP 2 614 767 A1**
(43) Date of publication of application: **17.07.2013**
(21) Application number: 11823414.5
(22) Date of filing: 24.08.2011
(51) Int. Cl.: A61B 1/04

(54) **IMAGE PICKUP DEVICE, ENDOSCOPE TIP SECTION PROVIDED WITH THE IMAGE PICKUP DEVICE, AND ENDOSCOPE TIP UNIT**

(30) Priority: 09.09.2010 JP 2010202302
(71) Applicant: Olympus Corporation, Shibuya-ku Tokyo 151-0072 (JP)
(72) Inventor: KOJIMA Kazuaki, Tokyo 151-0072 (JP)
(74) Representative: Winter, Brandl, Fürniss, Hübner, Röss, Kaiser, Polte - Partnerschaft
(86) International application number: PCT/JP2011/069082
(87) International publication number: WO 2012/032935

(57) **Abstract**

A distal end rigid member 11s, a prism 20 provided inside the distal end rigid member 11s and positioned at the rear in an optical axis direction of a lens for observing a site to be examined, an image pickup device 21 fixed to a light exit surface of the prism 20 inside the distal end rigid member 11 s, and a substrate 22 including a surface 22i fixed to the image pickup device 21 and another surface 22t fixed to an inner circumferential face 11sn of the distal end rigid member 11s are included, and the other surface 22t of the substrate 22 is formed in a shape that reduces or eliminates a space between the inner circumferential face 11sn of the distal end rigid member 11s and the other surface 22t of the substrate 22.

## Description

### Technical Field

The present invention relates to an image pickup apparatus that picks up an image of a site to be examined using a lens, a prism and an image pickup device, an endoscope distal end portion including the image pickup apparatus, and an endoscope distal end unit.

### Background Art

Conventionally, electronic endoscopes and electronic equipment such as camera-equipped mobile phones and digital cameras including an image pickup apparatus provided with a solid-state image pickup device (hereinafter simply referred to as image pickup device) such as a CCD and a CMOS have been known.

In general, a main part of an image pickup apparatus includes an image pickup device including a light-receiving section which an optical image of a site to be examined enters via a lens, a substrate electrically connected to the image pickup device, and the like. Video signals of the image of the site to be examined, which has been received by the image pickup device, are transmitted to the outside of the image pickup apparatus via a signal wire extending out from the substrate.

Here, where an image pickup apparatus is provided inside a distal end portion of an insertion portion of an endoscope, which is to be inserted into a site to be examined, ordinarily, inside the distal end portion, the image pickup device is arranged at a light-concentrating position at the rear in an optical axis direction (hereinafter simply referred to as the rear) of the lens along a radial direction of the distal end portion orthogonal to the optical axis direction.

However, a decrease in diameter of the distal end portion results in reduction in space inside the distal end portion. Accordingly, in order to arrange an image pickup device in a small space along a radial direction of a distal end portion, it is necessary to use a downsized one for the image pickup device itself, which reduces the area of the light-receiving section and thus, resulting in the problem of a decrease in light sensitivity.

In view of such problem, Japanese Patent Application Laid-Open Publication No. 10-148745 discloses a configuration inside a distal end portion of an insertion portion of an endoscope in which an optical image is made to enter a light-receiving surface of an image pickup device arranged along an optical axis direction on a light exit surface of a prism provided at the rear of a lens unit including a plurality of lenses, via the prism, and transmits electric signals to the outside of the image pickup apparatus via a signal wire extending out from a substrate connected to the image pickup device.

With such configuration, the image pickup device is arranged along the optical axis direction inside the distal end portion, an outer size of the image pickup device in a direction along the optical axis direction, which is irrelevant to a decrease in diameter of the distal end portion, can be increased, enabling a sufficient size of the light-receiving section to be secured. Accordingly, even though the diameter of the distal end portion is decreased, light sensitivity comparable to those of conventional techniques can be secured.

Here, in the configuration disclosed in Japanese Patent Application Laid-Open Publication No. 10-148745, inside the distal end portion, the lens unit and the prism are provided at positions on the outer side in a radial direction of the distal end portion relative to a center of a space inside the distal end portion, and because the image pickup device receives light reflected by the prism, the image pickup device is positioned at the center of the space inside the distal end portion. Note that the substrate and the signal wire are also positioned at the center of the space inside the distal end portion.

However, with such configuration, if the diameter of the distal end portion is decreased, the space inside the distal end portion is also reduced, and thus, the image pickup device, the substrate and the signal wire which are positioned at the center of the space inside the distal end portion easily cause interference with other components inside the distal end portion, resulting in the problem of difficulty in arranging the other components in order to avoid the interference.

In other words, a decrease in diameter of the distal end portion results in the problem of difficulty in mounting a plurality of components in high density inside the distal end portion. In other words, there is the problem that a decrease in diameter of the distal end portion is difficult if interference between the image pickup device, the substrate and the signal wire and the other components inside the distal end portion is taken into consideration.

Note that the aforementioned problem is applicable not only to distal end portions of endoscopes, but also to cases where an image pickup device is provided inside a limited space of electronic equipment.

The present invention has been made in view of the problem, and an object of the present invention is to provide an image pickup apparatus including a configuration enabling the image pickup apparatus to be compactly mounted inside a limited space of electronic equipment, thereby achieving downsizing of a part in which the image pickup apparatus is arranged, an endoscope distal end portion including the image pickup apparatus, and an endoscope distal end unit.

### Disclosure of the Invention

### Means for Solving the Problem

An image pickup apparatus according to an aspect of the present invention includes an image pickup apparatus for picking up an image of a site to be examined, the apparatus including: a barrel body; a prism provided inside the barrel body and positioned at a rear in an optical axis direction of a lens for observing the site to be examined; an image pickup device fixed to a light exit surface of the prism inside the barrel body; and a substrate including a surface fixed to the image pickup device and another surface fixed to an inner circumferential face of the barrel body, wherein the other surface of the substrate is formed in a shape that reduces or eliminates a space between the inner circumferential face of the barrel body and the other surface of the substrate.

Also, in an endoscope distal end portion including an image pickup apparatus according to an aspect of the present invention, the image pickup apparatus according to the aspect is provided in an inner portion thereof.

Furthermore, an endoscope distal end unit according to an aspect of the present invention includes an endoscope distal end unit provided inside a distal end portion on a distal end side in an insertion direction of an insertion portion of an endoscope to be inserted into a site to be examined, wherein a plurality of body blocks each having a fan-like shape in a cross-section in a radial direction of the distal end portion and being formed in a columnar shape along the insertion direction are provided adjacent to one another along a circumferential direction of the insertion portion so that a cross-section in the radial direction of the distal end portion has a round shape; and wherein an image pickup apparatus that picks up an image of the site to be examined is provided inside any one block from among the plurality of body blocks.

### Brief Description of the Drawings

Fig. 1 is a partial perspective diagram illustrating a distal end side of an insertion portion of an endoscope with an image pickup apparatus according to the present embodiment provided in a distal end portion of the insertion portion;
Fig. 2 is a cross-sectional view of the distal end portion along line II-II in Fig. 1;
Fig. 3 is an enlarged perspective diagram illustrating the image pickup apparatus in Fig. 2;
Fig. 4 is a cross-sectional view of the distal end portion along line IV-IV in Fig. 2;
Fig. 5 is a plan view of the image pickup apparatus in Fig. 2 as viewed in a V-direction in Fig. 2;
Fig. 6 is a cross-sectional diagram illustrating a modification of a distal end portion in which the distal end rigid member in Fig. 4 is formed in a ring shape and an image pickup unit block including an image pickup apparatus 50 is provided in an inner space;
Fig. 7 is a partial perspective diagram illustrating a distal end side of an insertion portion of an endoscope in which an endoscope distal end unit is provided in a distal end portion of the insertion portion;
Fig. 8 is a cross-sectional view of the distal end unit of the distal end portion along line VIII-VIII in Fig. 7; and
Fig. 9 is a cross-sectional view of the distal end portion along line IX-IX in Fig. 8.

### Best Mode for Carrying Out the Invention

An embodiment of the present invention will be described below with reference to the drawings. It should be noted that the drawings are schematic ones, relationships between a thickness and a width of each member, ratios in thickness between the respective members and the like are different from actual ones, and it should be understood that the drawings include parts with dimensional relationships and/or ratios mutually different among the drawings. A description will be provided below taking a case where an image pickup apparatus is provided inside a distal end portion of an insertion portion of an endoscope as an example.

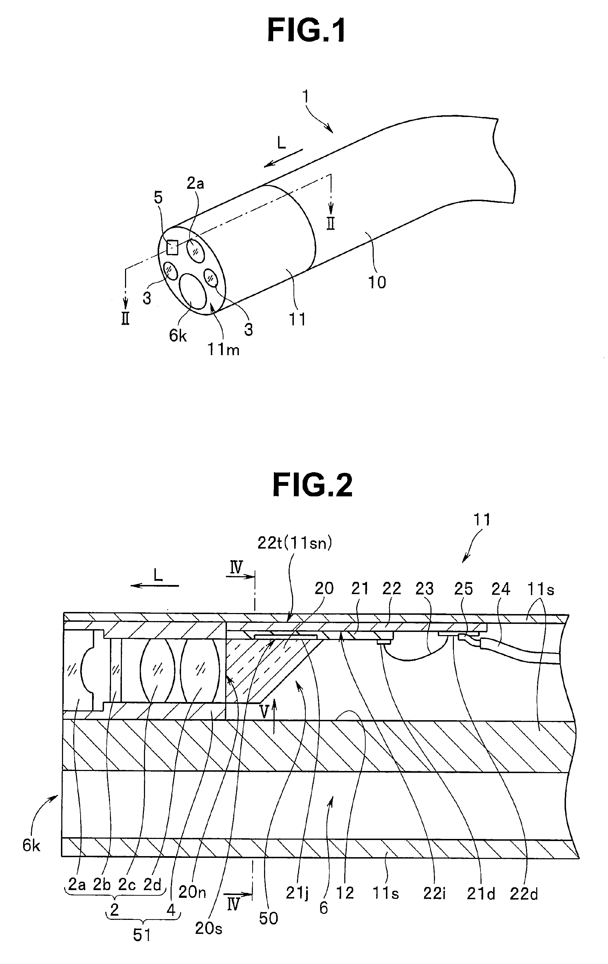

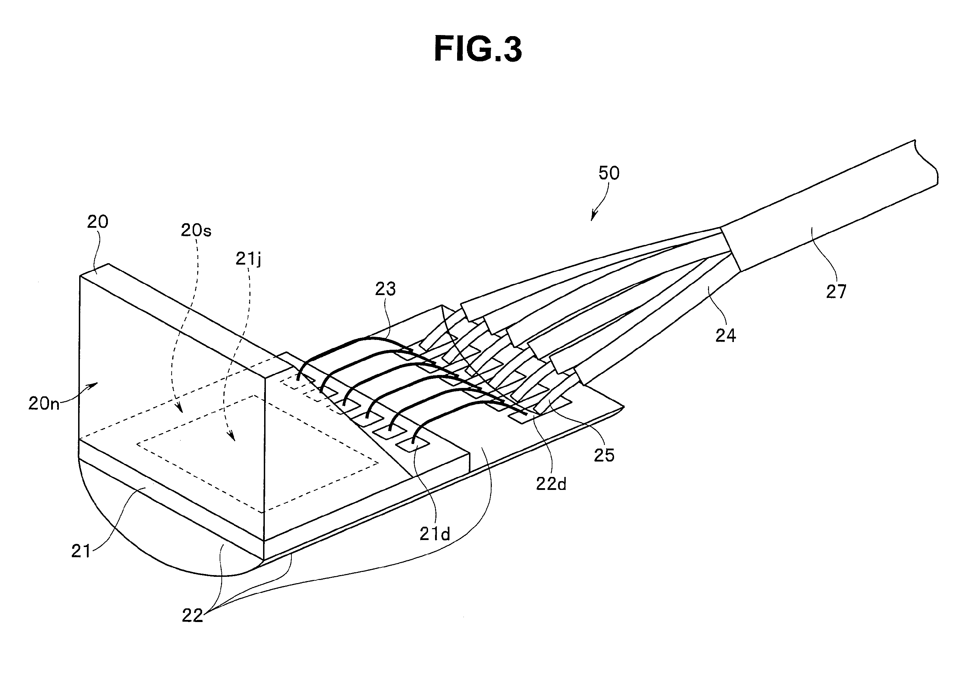

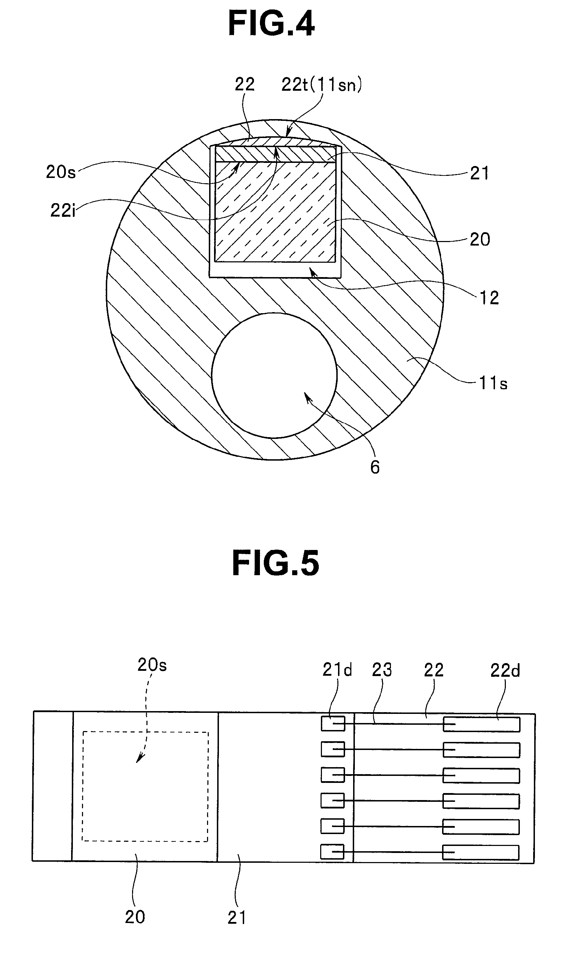

Fig. 1 is a partial perspective diagram illustrating a distal end side of an insertion portion of an endoscope with an image pickup apparatus according to the present embodiment provided in a distal end portion of the insertion portion; Fig. 2 is a cross-sectional view of the distal end portion along line II- II in Fig. 1; Fig. 3 is an enlarged perspective diagram illustrating the image pickup apparatus in Fig. 2; Fig. 4 is a cross-sectional view of the distal end portion along line IV-IV in Fig. 2; and Fig. 5 is a plan view of the image pickup apparatus in Fig. 2 as viewed in a V-direction in Fig. 2.

As illustrated in Fig. 1, at a distal end face 11m of a distal end portion 11 positioned on the distal end side in an optical axis direction L of a later-described lens 2 (hereinafter simply referred to as the distal end side) of an insertion portion 10 of an endoscope 1, an objective lens 2a for observing a site to be examined, for example, two illumination lenses 3 that illuminate the site to be examined, an air/water feeding nozzle 5 that supplies a fluid to a surface of the objective lens 2a to remove dirt on the surface of the objective lens 2a, and a distal end opening 6k of a channel 6 provided inside the insertion portion 10, the channel 6 being used when a treatment instrument is supplied to the site to be examined or a liquid or a solid positioned at the site to be examined is sucked. Note that the count of the illumination lenses 3 is not limited to two.

Also, as illustrated in Fig. 2, inside the distal end portion 11, an image pickup apparatus 50 that takes an image of a site to be examined is provided.

More specifically, inside the distal end portion 11, a distal end rigid member 11s, which is a barrel body having a cylindrical shape along the optical axis direction L, is provided.

In a through-hole 12 formed along the optical axis direction L in the distal end rigid member 11s, a lens unit 51, and the image pickup apparatus 50 positioned at the rear of the lens unit 51 are provided.

Note that in Fig. 2, illustration of a distal end cover covering an outer circumferential face and a distal end face of the distal end rigid member 11s is omitted for simplification of the drawing.

Furthermore, as illustrated in Figs. 1 and 2, the through-hole 12 is formed in the distal end rigid member 11s along the optical axis direction L on the outer side in a radial direction of the distal end portion 11 relative to a center of the distal end portion 11, more specifically, at a position in the vicinity of an outer circumferential edge portion of the distal end portion 11.

Note that in the distal end rigid member 11s, a plurality of through-holes in which, e.g., the above-described channel 6, non-illustrated illumination apparatuses including the above-described illumination lenses 2 and a fluid supply conduit that supplies a fluid to the above-described air/water feeding nozzle 5 are provided are each formed along the optical axis direction L, in addition to the through-hole 12.

A main part of the lens unit 51 includes the lens 2 for observing a site to be examined, the lens 2 including a plurality of lenses 2a to 2d, and a lens barrel 4 that holds the lens 2.

As illustrated in Figs. 2 to 5, a main part of the image pickup apparatus 50 includes a prism 20 arranged at a light-concentrating position for the lens 2d, an image pickup device 21 fixed to the prism 20 so that at least a light-receiving section 21j faces a light exit surface 20s of the prism 20, a substrate 22 including a surface 22i fixed to the image pickup device 21, and another surface 22t fixed to an inner circumferential face 11sn formed by the through-hole 12 of the distal end rigid member 11s, and a signal wire 27 (see Fig. 3) inserted inside the endoscope 1, the signal wire 27 extending out from terminals 22d of the substrate 22. Note that in Fig. 5, illustration of the signal wire 27 is omitted.

Here, as illustrated in Figs. 2 and 4, the distal end rigid member 11s including the inner circumferential face 11sn to which the substrate 22 is fixed, as described above, is formed so as to have a small thickness, for example, around 0.15 mm since the through-hole 12 of the distal end rigid member 11s is formed along the optical axis direction L at the position in the vicinity of the outer circumferential edge portion of the distal end portion 11.

Although it is desirable that the thickness be small to the extent possible in order to secure a large space inside the through-hole 12 to the extent possible, the thickness is set as a thickness providing a mechanical strength enough to keep from breakage in order to prevent breakage.

Also, as illustrated in Fig. 4, the inner circumferential face 11sn is formed in a circular arc shape since a region of the through-hole 12, the region forming in the inner circumferential face 11sn, is formed in a circular arc shape. Therefore, the inner circumferential face 11sn is naturally formed in a shape other than a circular arc shape depending on the shape of the region of the through-hole 12, the region forming the inner circumferential face 11sn.

The prism 20 is provided to change a direction of light falling on an incident surface 20n from the lens 2d so that the light enters the light-receiving section 21j of the image pickup device 21 from the exit surface 20s.

As illustrated in Fig. 3, the light-receiving section 21j of the image pickup device 21 is covered by the exit surface 20s of the prism 20 in a planar view, and a plurality of terminals 21d in a region of the image pickup device 21 other than a region of the prism 20 of the image pickup device 21 to which the prism is fixed are electrically connected one by one to a plurality of terminals 22d via lead wires 23, the count of the terminals 22d being equal to the count of the terminals 21d of the substrate 22.

As illustrated in Figs. 2 and 3, core wires 25 of a plurality of lead wires 24 extending out from a distal end side of the signal wire 27, the count of the lead wires 24 being equal to the count of the terminals 22d, are electrically connected one by one to the respective terminals 22d formed in a region of the substrate 22 other than a region of the substrate 22 to which the image pickup device 21 is fixed.

Consequently, video signals outputted from the image pickup device 21 are transmitted to the outside of the endoscope 1 via the terminals 21d, the lead wires 23, the terminals 22d, the core wires 25, the lead wires 24 and the signal wire 27.

The substrate 22 includes, e.g., glass epoxy or ceramic, and as illustrated in Figs. 3 and 4, the other surface 22t, which is a surface fixed to the inner circumferential face 11sn, is formed so as to have a shape conforming to the inner circumferential face 11sn, more specifically, a circular arc shape.

Consequently, the substrate 22 is arranged along the optical axis direction L inside the through-hole 12 in snug contact with the inner circumferential face 11sn. Furthermore, the image pickup device 21 fixed to the other surface 22i of the substrate 22 is also positioned on the inner circumferential face 11sn side relative to a center of the through-hole 12 inside the through-hole 12.

Here, a shape of the other surface 22t of the substrate 22 only needs to conform to a shape of the inner circumferential face 11sn. In other words, where the other surface 22t is fixed to the inner circumferential face 11sn, the fixation is achieved by making the shape of the other surface 22t and the shape of the inner circumferential face 11sn correspond to each other to bring the other surface 22t and the inner circumferential face 11sn into close contact with each other.

This is because if the other surface 22t of the substrate 22 is formed in a linear shape despite the inner circumferential face 11sn being formed in a circular arc shape, it is difficult to fix the other surface 22t to the inner circumferential face 11sn having a circular arc shape and a dead space is generated between the inner circumferential face 11sn and the other surface 22t, resulting in difficulty in mounting a plurality of components in high density inside the through-hole 12 when a diameter of the distal end portion 11 is decreased.

However, the shape of the other surface 22t does not need to perfectly correspond to the shape of the inner circumferential face 11sn, and may be any shape as long as the shape eliminates the aforementioned dead space.

In other words, even where the inner circumferential face 11sn has a circular arc shape while the other surface 22t has a linear shape, if a width in a width direction, i.e., a lateral direction in Fig. 4, of the other surface 22t is small and the substrate 22 has a cross-sectional shape having a width decreasing toward the inner circumferential face 11sn, a resulting dead space can be ignored virtually.

More specifically, the substrate 22 has a cross-section of a trapezoidal shape having a width decreasing toward the inner circumferential face 11sn and the other surface 22t forms an upper side of the trapezoidal shape with the decreased width, or if the substrate 22 has a cross-section of a shape having a width decreasing toward the inner circumferential face 11sn in which opposite ends in the width direction each have a stepped shape while the other surface 22t is formed in a linear shape with a small width, the dead space can be eliminated even though the shape of the other surface 22t does not correspond to the shape of the inner circumferential face 11sn.

Furthermore, the substrate 22 may include, for example, a multilayer substrate including multiple layers formed by depositing an insulator and a wiring pattern in a plurality of layers.

Furthermore, even if the other surface 22t of the substrate 22 is formed in a shape conforming to the inner circumferential face 11 sn, there is no influence in handling capability of the image pickup apparatus 50.

As described above, the present embodiment indicates that inside the through-hole 12 of the distal end rigid member 11s, the other surface 22t of the substrate 22 is formed in a shape conforming to the inner circumferential face 11sn of a thin portion formed by the through-hole 12 and the other surface 22t is fixed in close contact with the inner circumferential face 11sn.

Accordingly, as well as the substrate 22 can be positioned in close contact with the inner circumferential face 11sn along the optical axis direction L on the outer side in a radial direction of the through-hole 12 with no dead space generated between the other surface 22t and the inner circumferential face 11 sn, the signal wire 27 electrically connected to the terminals 22d of the substrate 22 can also be inserted in the vicinity of the inner circumferential face 11sn along the optical axis direction L, and furthermore, the image pickup device 21 can also be positioned on the inner circumferential face 11sn side relative to the center of the through-hole 12 inside the through-hole 12.

Accordingly, inside the through-hole 12, it is possible to make it difficult for the image pickup device 21, the substrate 22 and the signal wire 27 to come into contact with other components arranged inside the through-holes, enabling a plurality of components to be mounted in high density inside the through-hole 12. Note that this effect is especially significant where a diameter of the through-hole 12 is decreased along with a decrease in diameter of the distal end portion 11.

Also, when the image pickup device 21 is arranged inside the through-hole 12, it is only necessary that the substrate 22 be fixed in conformity to the shape of the inner circumferential face 11 sn, enabling enhancement in assemblability of the image pickup device 21.

Furthermore, while if the diameter of the through-hole 12 is decreased along with a decrease in diameter of the distal end portion 11, a space in the radial direction of the through-hole 12 is also reduced, which gives rise to a need to use a thinned image pickup device 21, with the present configuration, the image pickup device 21 is fixed to the substrate 22, enabling a mechanical strength of the thinned image pickup device 21 to be enhanced by the substrate 22.

According to the above, the image pickup apparatus 50 including a configuration enabling the image pickup apparatus 50 to be compactly mounted inside a limited space of the distal end portion 11, thereby downsizing of the distal end portion 11 in which the image pickup apparatus 50 is arranged, and the endoscope distal end portion 11 including the image pickup apparatus 50 can be provided.

Although the present embodiment has been described taking a case where the image pickup apparatus 50 is arranged in the through-hole 12 formed along the optical axis direction L in the distal end rigid member 11s as an example, the present invention is not limited to this case, and effects similar to those of the present embodiment can also be provided if the present invention is applied to a configuration in which the distal end rigid member 11s is formed in, for example, a thin ring having a large space inside and, e.g., the image pickup apparatus 50, the above-described lens unit 51, the above-described illumination apparatuses, the above-described channel 6 and the above-described fluid supply conduit that supplies a fluid to the air/water feeding nozzle 5 are provided in the space.

In this case, where the configuration of the present embodiment is employed, even if the diameter of the distal end portion 11 is decreased, that is, the diameter of the space inside the tubular distal end rigid member is decreased, the image pickup device 21, the substrate 22 and the signal wire 27 in the image pickup apparatus 50 can easily be arranged in the space with no interference with the other members such as the illumination apparatuses and the fluid supply conduit.

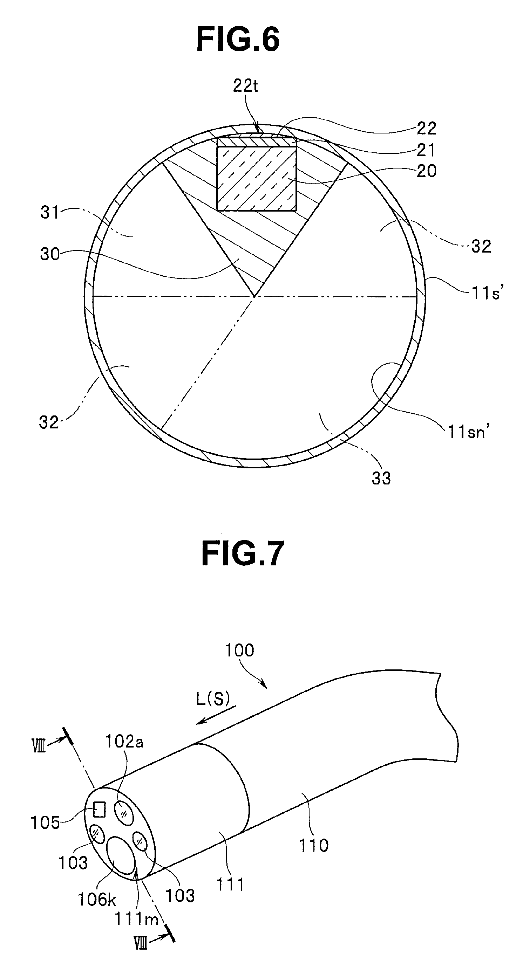

Another modification is described below with reference to Fig. 6. Fig. 6 is a cross-sectional diagram illustrating a modification of a distal end portion in which the distal end rigid member in Fig. 4 is formed in a ring shape and an image pickup unit block including the image pickup apparatus 50 is provided in an inner space.

As illustrated in Fig. 6, where the image pickup apparatus 50 is provided in an inner portion of a distal end rigid member 11s' formed in a ring shape, the present embodiment can be applied even to a configuration in which the image pickup apparatus 50 and the lens unit 51 are provided in an image pickup unit block 30 having a fan-like shape in a cross-section in a radial direction and having a columnar shape along an optical axis direction L and as with the present embodiment, the other surface 22t of the substrate 22 is brought into contact with and fixed to an inner circumferential face 11sn' of the distal end rigid member 11s'.

In this case, in the inner portion of the distal end rigid member 11s', in addition to the image pickup unit block 30, illumination unit blocks 32 including the above-described illumination apparatuses, a fluid supply unit block 31 including a fluid supply conduit, a channel unit block 33 provided with the channel 6 are fitted in one another so as to be adjacent to one another along a circumferential direction, the block units 30 to 33 each having a fan-like shape in a cross-section in the radial direction and having a columnar shape along the optical axis direction L as in the image pickup unit block 30, so that a round cross-section in the radial direction is provided by the plurality of block units 30 to 33.

Furthermore, although the present embodiment indicates that the image pickup apparatus 50 is provided inside the distal end portion 11 of the insertion portion 10 of the endoscope 1, the image pickup apparatus 50 may be provided in an inner portion of any of other electronic components, for example, a mobile phone. Even in such case, effects similar to those of the present embodiment can be provided as long as the other surface 22t of the substrate 22 is formed in a shape conforming to an inner circumferential face of a barrel body of the mobile phone.

### [Appendices]

As described in detail above, an embodiment of the present invention enables provision of a configuration such as stated below.
(1) An endoscope distal end unit provided inside a distal end portion on a distal end side in an insertion direction of an insertion portion of an endoscope to be inserted into a site to be examined,
   wherein a plurality of body blocks each having a fan-like shape in a cross-section in a radial direction of the distal end portion and being formed in a columnar shape along the insertion direction are provided adjacent to one another along a circumferential direction of the insertion portion so that a cross-section in the radial direction of the distal end portion has a round shape.
(2) The endoscope distal end unit according to appendix 1, wherein the plurality of body blocks include each of: an image pickup unit block in which an image pickup apparatus that picks up an image of the site to be examined and a lens unit for observing the site to be examined are provided; an illumination unit block in which an illumination apparatus that illuminates the site to be examined is provided; a fluid supply unit block in which a fluid supply conduit that supplies a fluid to the site to be examined or a lens in the image pickup apparatus is provided; and a channel unit block in which a channel used when a treatment instrument is supplied to the site to be examined or a liquid or a solid positioned at the site to be examined is sucked is provided.
(3) The endoscope distal end unit according to appendix 1 or 2, wherein the plurality of body blocks are joined to one another in the circumferential direction via joining portions provided at the respective body blocks.
(4) The endoscope distal end unit according to any one of appendices 1 to 3, wherein an outer circumferential face of the plurality of body blocks provided adjacent to one another along the circumferential direction so that the cross-section in the radial direction of the distal end portion has a round shape is covered by a fixing member that fixes the plurality of body blocks.

Conventionally, electronic endoscopes including an image pickup apparatus provided with a solid-state image pickup device (hereinafter simply referred to as image pickup device) such as a CCD and a CMOS have been known.

In general, a main part of an image pickup apparatus includes an image pickup device including a light-receiving section an optical image of a site to be examined enters via a lens, a substrate electrically connected to the image pickup device, and the like. Video signals of the image of the site to be examined, which has been received from the image pickup device, are transmitted to the outside of the image pickup apparatus via a signal wire extending out from the substrate.

Here, in an endoscope, an image pickup apparatus is provided at the rear of a lens unit inside a distal end portion on a distal end side in an insertion direction of an insertion portion to be inserted into a site to be examined.

Also, inside the distal end portion, e.g., illumination apparatuses that illuminate the site to be examined, a fluid supply unit that supplies a fluid to the site to be examined or the lens unit, and a channel used when a treatment instrument is supplied to the site to be examined or a liquid or a solid positioned at the site to be examined is sucked are provided, in addition to the image pickup apparatus.

More specifically, as disclosed in Japanese Patent Application Laid-Open Publication No. 2005-304585, an endoscope distal end portion has a configuration in which a distal end rigid member included in a distal end portion includes a plurality of through-holes formed along an optical axis direction of a lens and in each of the through-holes, any of, e.g., an image pickup unit including a lens unit and an image pickup apparatus, an illumination apparatus, a fluid supply unit and a channel is provided. In other words, an endoscope distal end portion has a configuration in which, e.g., an image pickup unit, an illumination apparatus, a fluid supply unit and a channel are integrally formed in a distal end rigid member.

However, if a diameter of the distal end portion is decreased or another component is further provided inside the distal end portion, a plurality of members are provided intricately in high density in the distal end rigid member, resulting in the problem of difficulty in assembling the plurality of members to the distal end rigid member, e.g., the plurality of members interfere with each other, and there also arises the problem of difficulty in taking the respective members out of the distal end rigid member at the time of repair. Furthermore, since the distal end rigid member also has a complicated shape, there is the problem that when the distal end rigid member is downsized, processing to provide the distal end rigid member becomes difficult, resulting in an increase in manufacturing costs.

The present appendices have been provided in view of the above problems, and an object of the present appendices is to provide an endoscope distal end unit, the endoscope distal end unit having a configuration enabling a plurality of members to be easily assembled to the inside of a distal end portion of an insertion portion of an endoscope in high density, enabling the plurality of members to be easily taken out for enhancement in repair capability, and also enabling reduction in manufacturing costs.

A configuration that achieves the above object will be described below with reference to Figs. 7 to 9.

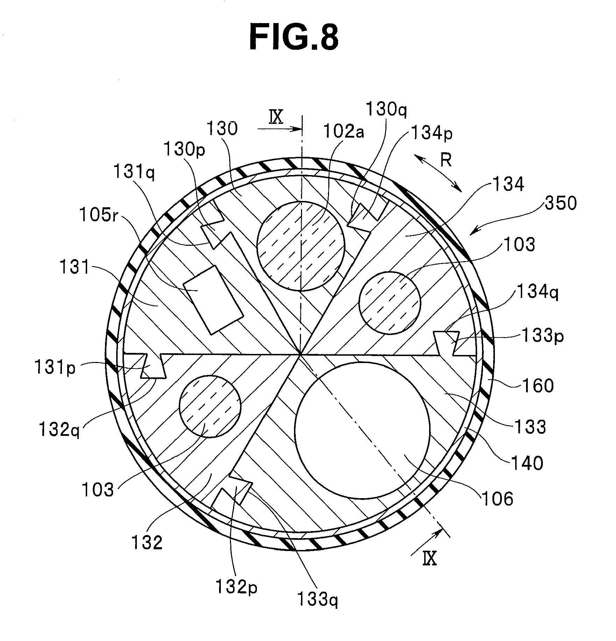

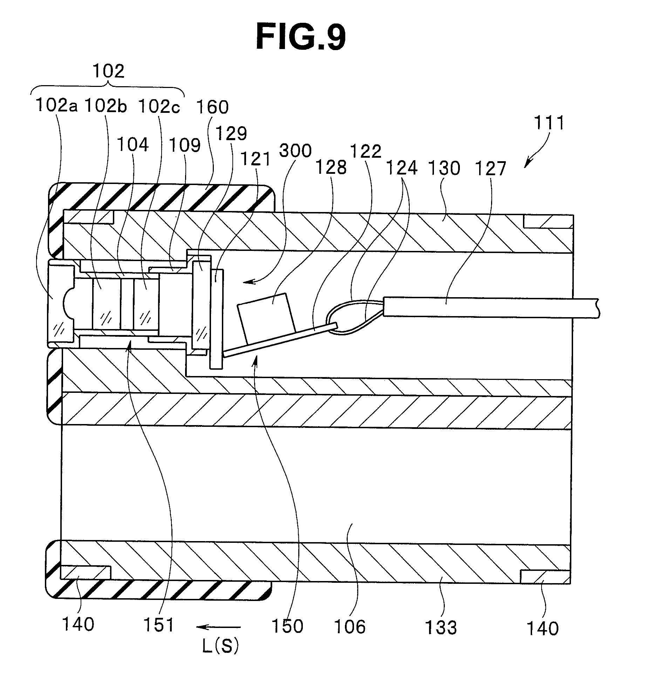

Fig. 7 is a partial perspective diagram illustrating a distal end side of an insertion portion of an endoscope in which an endoscope distal end unit according to the present appendices is provided in a distal end portion of an insertion portion; Fig. 8 is a cross-sectional diagram of a distal end unit of the distal end portion along line VIII-VIII of Fig. 7; and Fig. 9 is a cross-sectional diagram of the distal end portion along line IX-IX in Fig. 8.

As illustrated in Fig. 7, at a distal end face 111m of a distal end portion 111 in the distal end side in an insertion direction S (hereinafter simply referred to as the distal end side) of an insertion portion 110 of an endoscope 100, an objective lens 102a for observing a site to be examined, for example, two illumination lenses 103 that illuminate the site to be examined, an air/water feeding nozzle 105 that supplies a fluid to a surface of the objective lens 102a to remove dirt on the surface of the objective lens 102a, and a distal end opening 106k of a channel 106 provided inside the insertion portion 110, the channel 106 being used when a treatment instrument is supplied to the site to be examined or a liquid or a solid positioned at the site to be examined is sucked are provided. At the distal end face 111m, a forward water feed nozzle that supplies a fluid toward the site to be examined may be provided.

Furthermore, as illustrated in Fig. 9, inside the distal end portion 111, an image pickup unit 300 that picks up an image of a site to be examined is provided.

The image pickup unit 300 includes a lens unit 151 including a plurality of lenses 102a to 102c and a lens barrel 104 that holds the plurality of lenses 102a to 102c, and an image pickup apparatus 150.

The image pickup apparatus 150 includes a device barrel 109 including a distal end fixed to a proximal end side in an optical axis direction L of the lens barrel 104 (hereinafter simply referred to as the proximal end side), a cover glass 129 provided inside the device barrel, an image pickup device 121 fixed to the cover glass 129, a substrate 122 with an electronic component 128 mounted thereon, the substrate 122 being electrically connected to the image pickup device 121, and a signal wire 127 electrically connected to non-illustrated terminals of the substrate 122 via lead wires 124.

More specifically, inside the distal end portion 111, as illustrated in Fig. 8, a plurality of body blocks 130 to 134 each formed in a columnar shape along the insertion direction S and each having a fan-like shape in a cross-section in a radial direction of the distal end portion 111 are provided adjacent to one another along a circumferential direction R of the insertion portion 110 so that a cross-section in the radial direction of the distal end portion 111 has a round shape.

The body block 130 is an image pickup unit block with the image pickup apparatus 150 and the lens unit 151 provided therein, and each of the body blocks 132 and 134 is an illumination unit block with an illumination apparatus provided therein, the illumination apparatus including the illumination lens 103 that illuminates a site to be examined.

Furthermore, the body block 131 is a fluid supply unit block with a fluid supply conduit 105r provided therein, a distal end of the fluid supply conduit 105r including the air/water feeding nozzle 105 that supplies a fluid to the site to be examined or the lens 102a of the image pickup apparatus 150, and the body block 133 is a channel unit block provided with the channel 106 used when a treatment instrument is supplied to the site to be examined or a liquid or a solid positioned at the site to be examined is sucked.

Accordingly, the plurality of body blocks 130 to 134 are provided inside the distal end portion 111, whereby the objective lens 102a, for example, two illumination lenses 103, the air/water feeding nozzle 105, and the distal end opening 106k of the channel 106 are provided at the distal end face 111m while the image pickup unit 300, the illumination apparatuses, the fluid supply conduit 105r and the channel 106 are provided inside the distal end portion 111.

Furthermore, as illustrated in Fig. 8, the respective body blocks 130 to 134 are joined to one another in the circumferential direction R via joining portions 130p to 134p provided at the respective fixed body blocks 130 to 134 and then fixed by later-described fixing members 140.

Outer circumferences of the fixing members 140 and the respective body blocks 130 to 134 are covered by a distal end cover 160. What covered by the distal end cover 160 provides the distal end portion 111.

More specifically, the body block 130 is joined to the body block 131 in the circumferential direction R as a result of the joining portion 130p being fitted in a groove 131q, and the body block 131 is joined to the body block 132 in the circumferential direction R as a result of the joining portion 131p being fitted in a groove 132q. Furthermore, the body block 132 is joined to the body block 133 in the circumferential direction R as a result of the joining portion 132p being fitted in a groove 133q, the body block 133 is joined to the body block 134 in the circumferential direction R as a result of the joining portion 133p being fitted in a groove 134q, and the body block 134 is joined to the body block 130 in the circumferential direction R as a result of the joining portion 134p being fitted in a groove 130q.

Also, as illustrated in Figs. 8 and 9, each of a distal end side and a proximal end side of the outer circumferential faces of the plurality of body blocks 130 to 134 joined adjacent to one another along the circumferential direction R so that the cross-section in the radial direction of the distal end portion 111 has a round shape is covered by a fixing member 140 fixing the plurality of body blocks 130 to 134 joined.

As illustrated in Fig. 9, the fixing members 140 are fitted in respective grooves formed at the distal end side and the proximal end side of the plurality of body blocks 130 to 134.

Accordingly, use of the fixing members 140 eliminates the need to use the joining portions 130p to 134p for fixing the respective blocks 130 to 134 to one another.

Furthermore, as illustrated in Figs. 8 and 9, at outer circumferential faces of the fixing members 140 and the plurality of body blocks 130 to 134 and the distal end faces of the body blocks 130 to 131 are covered by the distal end cover 160.

The plurality of body blocks 130 to 134, the fixing member 140 and the distal end cover 160 provide a distal end unit 350 with the present configuration.

With such configuration, when the image pickup unit 300, the illumination apparatuses, the fluid supply conduit 105r and the channel 106 are provided inside the distal end portion 111, mere provision of the distal end unit 350 inside the distal end portion 111, that is, the plurality of body blocks 130 to 134 are merely fitted in one another to join the respective blocks 130 to 134 to one another in the circumferential direction R via the joining portions 130p to 134p and then covered by the fixing members 140 and the distal end cover 160, enabling easy integral assembling of the image pickup unit 300, the illumination apparatuses, the fluid supply conduit 105r and the channel 106.

Furthermore, for example, if the image pickup apparatus 150 fails, the image pickup apparatus 150 can easily be exchanged by removing only the body block 130 from the inside of the distal end portion 111, ensuring enhancement in exchange capability compared to the conventional techniques.

Furthermore, the body blocks 130 to 134 each have a simple shape such as a fan-like shape in a cross-section of the radial direction and a columnar shape in the insertion direction, and thus, costs for processing to provide the respective blocks 130 to 134 can be reduced compared to conventional one-piece body blocks (distal end rigid members) having a complicated processed shape.

Furthermore, the image pickup unit 300, the illumination apparatuses, the fluid supply conduit 105r and the channel 106 are provided inside the respective blocks 130 to 134, and thus, the respective members do not interfere with one another, and in addition, even if a diameter of the distal end portion 111 is decreased, it is only necessary to downsize the respective blocks 130 to 134, enabling a plurality of components to be mounted in high density inside the distal end portion 11.

According to the above, an endoscope distal end unit 350 is provided having a configuration enabling a plurality of members to be easily assembled in high density inside a distal end portion 111 of an insertion portion 110 of an endoscope 100, enabling the plurality of members to be easily removed for enhancement in repair capability, and also enabling reduction in manufacturing costs.

Furthermore, the above-described embodiment includes inventions of various phases, and a proper combination of a plurality of elements disclosed herein enables extraction of various inventions. For example, even if several elements are deleted from all the elements indicated in the above embodiment, a resulting configuration with the elements detected may be extracted as an invention as long as such configuration can solve the problem stated in the Problems to be Solved by the Invention section and provide effects described in the Advantages of the Invention.

The present application claims the priority of Japanese Patent Application No. 2010-202302 filed in Japan on September 9, 2010, the disclosure of which is incorporated in the description, the claims and the drawings in the present application by reference.

## Claims

1. An image pickup apparatus for picking up an image of a site to be examined, the apparatus comprising:
a barrel body;
a prism provided inside the barrel body and positioned at a rear in an optical axis direction of a lens for observing the site to be examined;
an image pickup device fixed to a light exit surface of the prism inside the barrel body; and
a substrate including a surface fixed to the image pickup device and another surface fixed to an inner circumferential face of the barrel body,
wherein the other surface of the substrate is formed in a shape that reduces or eliminates a space between the inner circumferential face of the barrel body and the other surface of the substrate.

2. The image pickup apparatus according to claim 1, wherein the substrate includes a multilayer substrate including an insulator and a wiring pattern deposited in a plurality of layers.

3. The image pickup apparatus according to claim 1 or 2, wherein the other surface of the substrate is formed in a shape conforming to the inner circumferential face of the barrel body.

4. The image pickup apparatus according to claim 3, wherein the other surface of the substrate is formed in a shape that comes into close contact with the inner circumferential face.

5. The image pickup apparatus according to any one of claims 1 to 4, wherein the prism, the image pickup device and the substrate are arranged inside a through-hole formed in a member inside the barrel body, the through-hole including a part forming the inner circumferential face of the barrel body, the through-hole being positioned on the inner circumferential face side relative to a center of the barrel body in a radial direction of the barrel body.

6. The image pickup apparatus according to any one of claims 1 to 4,
wherein the barrel body is formed in a ring shape including a space in an inner portion thereof, the inner portion forming the inner circumferential face of the barrel body; and
wherein the prism, the image pickup device and the substrate are arranged on the inner circumferential face side in the space relative to the center of the barrel body in a radial direction of the barrel body.

7. The image pickup apparatus according to claim 6, wherein the prism, the image pickup device and the substrate are provided in a block positioned inside the space.

8. An endoscope distal end portion including an image pickup apparatus, wherein the image pickup apparatus according to any one of claims 1 to 7 is provided in an inner portion thereof.

9. An endoscope distal end unit provided inside a distal end portion on a distal end side in an insertion direction of an insertion portion of an endoscope to be inserted into a site to be examined,
wherein a plurality of body blocks each having a fan-like shape in a cross-section in a radial direction of the distal end portion and being formed in a columnar shape along the insertion direction are provided adjacent to one another along a circumferential direction of the insertion portion so that a cross-section in the radial direction of the distal end portion has a round shape; and
wherein an image pickup apparatus that picks up an image of the site to be examined is provided inside any one block from among the plurality of body blocks.

10. The endoscope distal end unit according to claim 9,
wherein the plurality of body blocks include each of:
an image pickup unit block in which the image pickup apparatus and a lens unit for observing the site to be examined are provided;
an illumination unit block in which an illumination apparatus that illuminates the site to be examined is provided;
a fluid supply unit block in which a fluid supply conduit that supplies a fluid to the site to be examined or to a lens in the image pickup apparatus is provided; and
a channel unit block in which a channel used when a treatment instrument is supplied to the site to be examined or a liquid or a solid positioned at the site to be examined is sucked is provided.

11. The endoscope distal end unit according to claim 9 or 10, wherein the plurality of body blocks are joined to one another in the circumferential direction via joining portions provided at the respective body blocks.

12. The endoscope distal end unit according to any one of claims 9 to 11, wherein an outer circumferential face of the plurality of body blocks provided adjacent to one another along the circumferential direction so that the cross-section in the radial direction of the distal end portion has a round shape is covered by a fixing member that fixes the plurality of body blocks.

## Amended claims

### Amended claims under Art. 19.1 PCT

**1.** An image pickup apparatus for picking up an image of a site to be examined, the apparatus comprising:
a barrel body;
a prism provided inside the barrel body and positioned at a rear in an optical axis direction of a lens for observing the site to be examined;
an image pickup device fixed to a light exit surface of the prism inside the barrel body; and
a substrate including a surface fixed to the image pickup device and another surface fixed to an inner circumferential face of the barrel body,
wherein the other surface of the substrate is formed in a shape that reduces or eliminates a space between the inner circumferential face of the barrel body and the other surface of the substrate.

**2.** The image pickup apparatus according to claim 1, wherein the substrate includes a multilayer substrate including an insulator and a wiring pattern deposited in a plurality of layers.

**3.** The image pickup apparatus according to claim 1 or 2, wherein the other surface of the substrate is formed in a shape conforming to the inner circumferential face of the barrel body.

**4.** The image pickup apparatus according to claim 3, wherein the other surface of the substrate is formed in a shape that comes into close contact with the inner circumferential face.

**5.** The image pickup apparatus according to any one of claims 1 to 4, wherein the prism, the image pickup device and the substrate are arranged inside a through-hole formed in a member inside the barrel body, the through-hole including a part forming the inner circumferential face of the barrel body, the through-hole being positioned on the inner circumferential face side relative to a center of the barrel body in a radial direction of the barrel body.

**6.** The image pickup apparatus according to any one of claims 1 to 4,
wherein the barrel body is formed in a ring shape including a space in an inner portion thereof, the inner portion forming the inner circumferential face of the barrel body; and
wherein the prism, the image pickup device and the substrate are arranged on the inner circumferential face side in the space relative to the center of the barrel body in a radial direction of the barrel body.

**7.** The image pickup apparatus according to claim 6, wherein the prism, the image pickup device and the substrate are provided in a block positioned inside the space.

**8.** An endoscope distal end portion including an image pickup apparatus, wherein the image pickup apparatus according to any one of claims 1 to 7 is provided in an inner portion thereof.

**9.** (CANCEL)

**10.** (CANCEL)

**11.** (CANCEL)

**12.** (CANCEL)
